# EUROPEAN PATENT SPECIFICATION

(11) **EP 2 525 762 B1**
(45) Date of publication and mention of the grant of the patent: **11.12.2013**
(21) Application number: 11701737.6
(22) Date of filing: 19.01.2011
(51) Int. Cl.: A61H 35/02

(54) **HANDHELD APPARATUS FOR EYE-WASHING**
Tragbare Vorrichtung zur Augenspülung
Appareil portable pour le lavage des yeux

(30) Priority: 22.01.2010 US 297421 P; 22.01.2010 EP 10151399
(43) Date of publication of application: 28.11.2012
(73) Proprietor: First Aid Company ApS, 4040 Jyllinge (DK)
(72) Inventor: PEDERSEN, Steen H.H., DK-4040 Jyllinge (DK)
(74) Representative: Nielsen, Leif
(86) International application number: PCT/DK2011/050013
(87) International publication number: WO 2011/088833

(56) References cited:
- EP-A2- 0 070 128
- CN-Y- 201 094 731
- US-A- 4 131 115
- US-A- 4 543 096
- US-B2- 7 331 944

## Description

### Field of the Invention

The present invention relates to a handheld eye wash apparatus used for first aid where there is a need for immediate washing of the eye, caused by foreign objects or chemicals in and/or around the eye(s).

### Background of the Invention

Most foreign objects, especially acidic or alkaline substances and liquids entering the eye, causing a seizure condition in the eye closures, which greatly complicates the opening and thus washing eye. Accidents involving dangerous substances and liquids can cause great pain and damage to the eye and produce panic-like reactions in casualties, which in particular requires quick and effective first aid.

The most common known handheld disposable eye wash liquids consist primarily of a container (made of a soft plastic bottle or an aluminium cylinder under pressure) with a nozzle or nozzles. The container contains eye wash liquid, and exist with or without the attached eye cup or eye support.

The apparatus from the prior art for treatment of the eyes by liquids for ophthalmic and washing purposes can in relation to the present invention by divided into four groups:
1. Passive eyelid openers and apparatus for ophthalmic purposes
2. Active eyelid openers and dispensors for ophthalmic purposes
3. Eyecups and apparatus for eye washing purposes
4. Active eyelid openers and apparatus for eye washing purposes

### Passive eyelid openers and apparatus for ophthalmic purposes

US 6,336,917 and US 6,569,131 relate to an ocular eye treatment and inspection speculum, namely a metered spray eye mist apparatus and a method of operation therefore, respectively.

The ocular inspection and treatment apparatus comprises an adjustable outer housing including a peripheral edge for contacting the bony orbit surrounding the eye and an inner housing including a peripheral edge, the inner housing being concentrically disposed within the outer housing, for contacting and retracting the eyelids to expose the eye for inspection and treatment. One end of the inner housing is adapted to receive a dispenser for administering a metered spray of medicine or a lavage to the eye as it is held open by the ocular treatment apparatus.

The method of using the ocular apparatus for inspecting the eye or administering the medicine or lavage to the eye include adjusting the inner and outer housings, positioning the apparatus on the eye to have the inner housing contact the eyelids peri-ocularly to retract the eyelids and to have the outer housing contact the bony orbit periorbitally.

US 6,336,917 and US 6,569,131 disclose an apparatus and method for primarily ophthalmic purposes. The device comprises a passive eye opener and a metered spray with a vial for medication. The device is handheld, but not suitable for eye wash and neither for operating by the patient/ injured subject himself.

### Active eyelid openers and dispensors for ophthalmic puposes

US 4,543,096 relates to an eyedrop dispenser which includes a plastic squeeze bottle having a dispensing nozzle at one end. A collar overfits the dispenser end of the bottle and carries a pair of cooperating, forwardly extending fingers for eyelid contacting purposes. The fingers terminate forwardly in respective eyelid contactors and one finger is pivotally movable relative to the other to spread the eyelid contactors during the eyedrop dispensing process. The movable finger includes an operator extension which engages the bottle sidewall when the eyelid contactors are separated to squeeze the bottle and dispense the eyedrops.

US 5,064,420 relates to an eyelid opener useful for opening a person's eye in order to dispense an ophthalmic fluid into the eye.

The eyelid opener comprises a collar having internal lobes arranged to enable the collar to be screwed onto the threads of an ophthalmic bottle. A shoulder extends across the collar and limits the engagement of the collar on the bottle threads. The bottle tip passes through a hole in the shoulder.

To the collar are joined a pair of flexible wings. The two wings terminate in respective flanges sized and shaped to conform to substantially the length of the exteriors of the human eyelids.

The wings can be squeezed together and placed on the eyelids. Releasing the wings causing them to return to their undeflected configurations and thereby open the eye. Then a drop of fluid in the bottle can be dispensed into the open eye.

Both US 4,543,096 and US 5,064,420 disclose an active eyelid opener and application of liquids for ophthalmic purposes, which means that a given subject actively can opens his eye(s) and keep it open during the application of liquids. The dispensers are typically soft bottles, and the application of liquid in form of drops is accomplished by simple squeezing the bottles. Thus the dispensers have a limited ability and capacity for washing the eye, and neither of the documents describes a well defined simultaneous activation of opening the eyes and activation of the application of liquid.

US 7,331,944 discloses an ophthalmic dispenser with a rigid housing defining a fluid-receiving chamber, a flexible bladder receivable within the fluid-receiving chamber, a pump coupled in fluid communication with the fluid-receiving chamber, and a nozzle leaving a valve. The valve is coupled in fluid communication between the valve seat and the pump. The rigid housing is mountable within a cartridge which, in turn, is mountable within a dispenser housing including an eyelid depressor and a trigger for simultaneously actuating the eyelid depressor and pump.

Thus the dispenser is capable of depressing the one eyelid, but not both, and is thereby less efficient than the other active eye openers described in the present application. The pressurized fluid flow is obtained by activation of a pump, not by a pressurized container. Finally the dispenser is for ophthalmic purposes in metered doses and not eye washing according to the present invention.

### Eyecups and apparatus for eye washing imposes

US 5,201,726 relates to an eye-bathing device comprises an eye-cup adapted to receive the eye being bathed, and a pump for delivering an eye bathing solution to the eye-cup through a spray nozzle. The eye-cup has an inlet opening aligned with the spray nozzle. The pump is hand-operated and is actuated by a lever for delivering an eye-bathing solution from a separate reservoir connected to the pump, and is located within a housing supporting the eye-cup.

US 5,201,726 discloses a device for eye washing. The device comprises an eye cup, and the device as such is operable with one hand. However the portability of the device is limited due to the dependency of a separate reservoir for the liquid. Further the eye cup do not have any means for directing waste washing liquid away from the eye area and is not able to keep the eye open during the wash.

### Active eyelid openers and apparatus for eye washing purposes

EP 0 070 128 on which the preamble of claim 1 is based discloses an apparatus for eye washing, where the apparatus comprises two rocker arms ("blades"), which are hinged at a hinge, where each of the rocker arms on one side of the hinge has a jaw ("eyelid engaging mean") for placing on the eyelids, and a wing (handle) on the opposite side of the hinge, thereby by manual compression of the wings, and the thereby caused rotation of the rocker arms around the hinge, to force the jaws and thus the eyelids apart. A tubular valve body is disposed and supported between the rockerarms, and the valve body and is connected to a flexible tube provided with means for connection to a fluid source, for example, a water faucet. The valve body comprises adjustable valve means for manual adjusting the flow of fluid therethrough.

Thus EP 0 070 128 discloses an active eyelid opener and application of liquids for eye washing purposes, which means that a given subject actively can opens his eye(s) and keep it open during the application of liquids. However the activation of the valve is for manual predetermination and thereby not coupled to the compression of the wings. Further the only example on a liquid source is a water faucet.

In general the prior art hereby achieve the following different application methods;
1. Without eye cup / support: eye wash liquid is poured or sprayed directly into the eye through a container with a nozzle / nozzle using one hand. Eye kept open with the other hand thumb and forefinger.
2. With eye cup / Support: Eye cup / container support is deployed against the eye and head bent backwards, after which the liquid is poured / injected into the eye through a nozzle / nozzle using one hand. In most cases, the eye again kept open with the other hand thumb and forefinger.

As described above, it is in cases where foreign objects in the eye cause pain and/or seizure condition necessary to force the eye open by the injured person's own fingers or in most cases by use of a helping persons finger. This leads to a significantly increased risk of transmission to the eye and its surroundings of any finger-born foreign body and/or chemical.

In case of accident where there has been foreign objects in the one eye and the injured person "only" can orient themselves with the other eye, it is incredibly difficult to judge distances and make controlled movements. For method 1, where there exists an eye cup / support, it can be extremely difficult to make a safe and effective washing of the eye, because management of the container is reduced. Method 2 has a support device, which in most cases are closed (cup shaped) and which thus prevents the possibility that eye wash process can be visually inspected, since the eye is hidden by the cup. An Eye cup can not force the eye open and thus have only the function that it supports the container and directs the liquid toward the eye. Injured subjects will as a result of pain have a tendency to force the eye in the closed position, which encumbers washing process.

Furthermore, the known methods only allow the injured subject to provide first aid to himself in one eye at a time, as one hand holds the washing bottle and the other is used to keep the eye open.

In all cases, using "soft" containers, it is necessary to keep your head in a backwardleaning position of the liquid can be poured into the eye. The vast majority of the soft plastic containers also have the disadvantage that they are building a vacuum in the bottle during washing, which means that the process may be interrupted and starts again when the pressure is lifted.

In the first case of eye accidents with foreign objects and especially dangerous liquids and substances, there is a need for large amounts of eye wash liquid. There was previously developed an eye-opening device, and here refers to a known eye drop dispenser with eye-opening devices from U.S. Patent No. 4,543,096 and US 5,064,420.

The designs as they are known from U.S. Patent No. 4,543,096 and US 5,064,420 are intended, and most effective, where there is a need to bring small doses of medications for one kind or another to the eye. The known construction has several limitations. The design makes it suitable for use only on soft bottles, where it is possible to squeeze the bottle together and thereby create a pressure in the cylinder that forces the liquid to the eye. The design also makes it difficult to mount on the larger bottles / containers with "broad shoulders". The solution can only be used when the head held back, which restricts freedom of movement in case of accidents significantly. Further, for the solution to work, it is dependent on the support obtained by mounting on the eye wash container according to the invention.

In all accident situations, it is crucial to the outcome of injury prevention that washing is effective, i.e. quickly, accurately and abundantly. It is in all situations very appropriate that washing can be done with one hand so that the injured have an extra hand free for other purposes related to the accidental situation. Thus, washing of an eye, operating with one hand ensures a mobility that is important for the injured freedom to move around (for example, open doors, call for help or make other important things in order to ensure effective first aid). Additionally, in cases where required, the one hand operation can ensure that the injured person can perform a wash of both eyes simultaneously.

It is also appropriate that the washing process can commence immediately and independently of the injured person's physical position in the situation. It is therefore of great importance that washing can be performed independent from the injured person keeping his head in a certain position. In addition, during the washing process, it is important to minimize the risk of the entering of additional foreign objects or chemicals in and around the eye.

Even in EP 0 070 128 which is considered closest prior art, the activation of the valve and the eye lid opening is not directly coupled and not simultaneously. Further the liquid source is not a pressurized container. Thereby the apparatus is not suitable for one-hand operation and not for the injured person to move freely around.

In conclusion, none of the other cited documents give in combination with EP 0 070 128 any pointers to a solution eliminating the disadvantages of the prior art apparatus for eye washing.

### Object of the Invention

Thus the prior art do not disclose any handheld device for eye wash purposes, alone or in combination, comprising an active eye opener capable of the simultaneous initiation/activation of eye washing. Therefore the object of the present invention is to provide a solution that by use of one hand only can force an eye open, and simultaneously can wash the eye quickly, accurately and with plenty of liquid independent of orientation of the head and the injured subjects physical position.

More specifically, the object of the present invention is to:
1. Conduct the process of opening and washing the eye without direct contact with "unclean" fingers on the skin around the eye and thus eliminate the risk of introduction of additional foreign or dangerous substances / chemicals.
2. Hold the eye(s) open during the whole washing process.
3. Maintain a safe and proper distance between the eye and the nozzle during washing.
4. Lead the washing liquid directly into the eye, where it has the greatest effect.
5. Direct waste washing liquid unhindered away from the eye after washing.
6. Wash in more than 15 minutes without changing container / eye wash.
7. Watch the washing / treatment of the eye freely throughout the first aid process.
8. Allow the injured subject to wash both eyes simultaneously.
9. Wash the eye(s) independently of a specific physical orientation of the head of the injured person.

### Description of the Invention

To solve the problem an apparatus was developed constituting a combination of a apparatus for opening and activation of the other part of the combination, the spray container comprising eye wash liquid, where the eye opener mechanically force a closed eye open and simultaneously activates the spay container and thereby the immediate washing of the eye(s).

Accordingly the invention relates in a first aspect to an apparatus for eye washing, where the apparatus comprises two rocker arms , which are hinged at a hinge , where each of the rocker arms on one side of the hinge has a jaw for placing on the eyelids, and a wing on the opposite side of the hinge, thereby by manual compression of the wings, and the thereby caused rotation of the rocker arms around the hinge, to force the jaws and thus the eyelids apart, where the apparatus comprises a connecting part for connection to a liquid container. The rocker arms are connected to a pressure device configured by compression of the wings to press a pressure valve on the liquid container being a pressurized container of aerosol type with the pressure valve, and thereby cause the release of liquid from the liquid container.
The apparatus is suitable for operation with one hand by the manual compression of the wings when placing the jaws of the apparatus on the eyelids. Thus the apparatus is placed with the jaws on the eyelids; and the wings are compressed at each opposite side of the hinge, thereby causing the rotation of the rocker arms around the hinge to force the jaws and thus the eyelids apart, activating the valve to release the liquid from the container, and washing the eye. This operation of eye washing may be performed with one hand throughout.

### The new technical effect

The combination of mounting an eye-opening device on a pressurized container containing eye wash liquid ensures that critical first aid can be performed on oneself or others quickly, accurately and efficiently with one hand. The combination ensures that washing is safe, accurate and optimal in relation to the dosage of liquid and the time the eye must be washed and the injured person's mobility in the situation increased dramatically.

The invention comprises an apparatus, which is a combination of an eye-opener device mounted on a pressurized container containing sterile washing liquid, which together has the effect:
1. The injured eye can be forced open.
2. The injured eye is optimally open during a washing process without the risk of introduction of additional foreign and dangerous objects / chemicals.
3. Effective washing of an eye in a controlled direction.
4. A good view to the injured eye with the possibility to control the washing process.
5. The eye wash liquid is automatically added to the eye at the moment the eye is forced open.
6. There is an optimal dosage of liquid to the eye.
7. Washing can be performed for the required period (min. 15 minutes per container).

### Definitions

The term liquid can be any liquid, solution or fluid, suitable for a given eye wash purpose. One example is an isotone (0.9%) sterile sodium chloride solution.

### Description of the Drawing

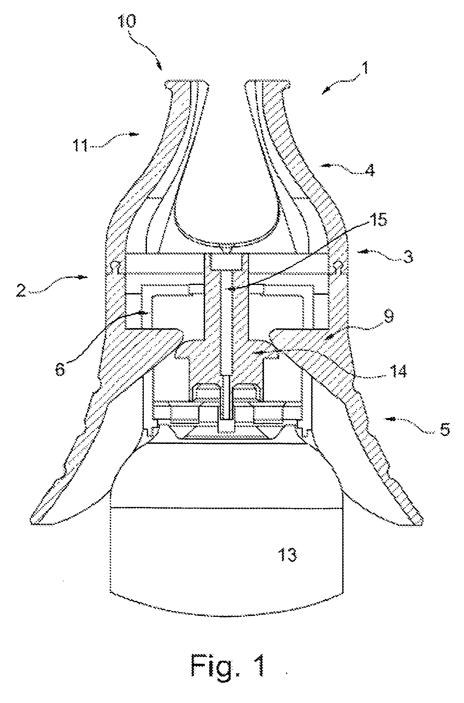

**Figure 1** is a cross sectional view of the apparatus combined with a liquid container of the aerosol type.

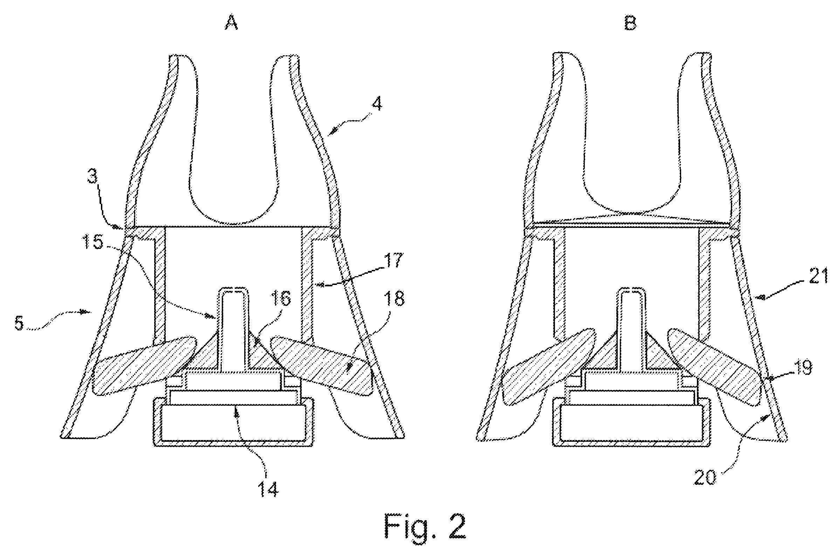

**Figure 2** is a cross sectional view of the apparatus. **A** is the apparatus with the wings in the uncompressed condition. **B** is the apparatus with the wings in the compressed condition.

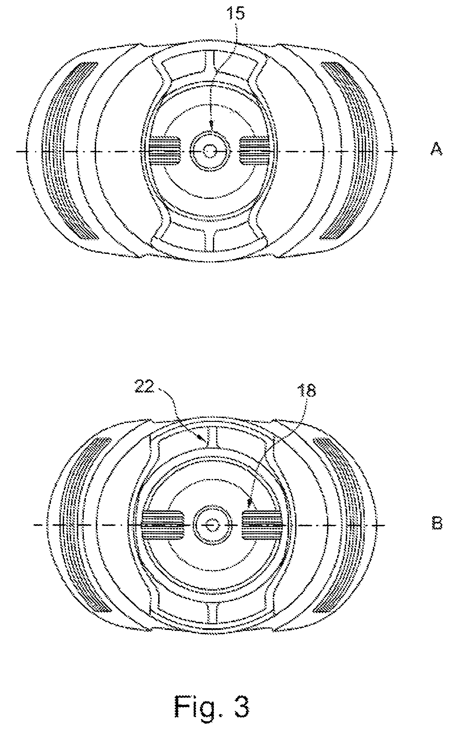

**Figure 3** is a top view of the apparatus. **A** is the apparatus with the wings in the uncompressed condition. **B** is the apparatus with the wings in the compressed condition.

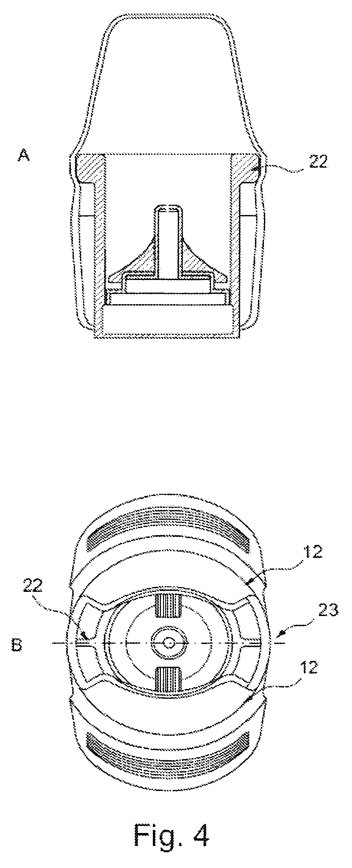

**Figure 4A** is a rotated cross sectional view of the apparatus. **Figure 4B** is a top view of the apparatus.

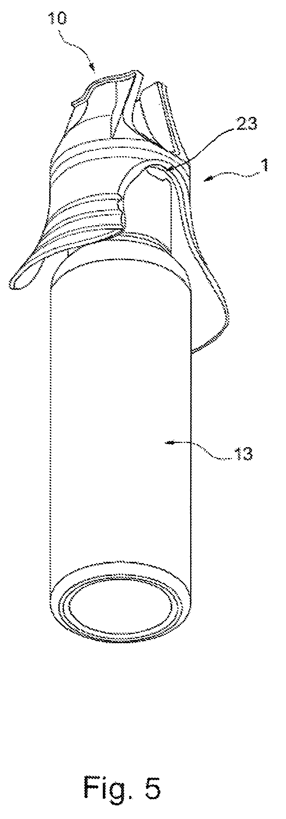

**Figure 5** is a perspective bottom view of the apparatus combined with a liquid container of the aerosol type.
**Figure 6** illustrated a method of eye washing using the apparatus combined with a liquid container of the aerosol type. **A** illustrates the placement of the apparatus on the eyelids and the initiation of compressing the wings. **B** illustrates the eye wash after compression of the wings.

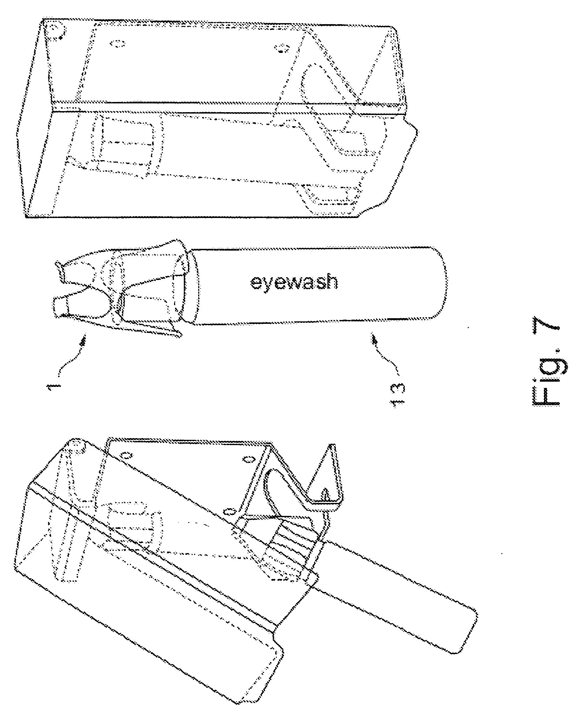

**Figure 7** is a perspective side view of the apparatus combined with a liquid container of the aerosol type and a device for storage of the combination.

### Detailed Description of the Invention

The present invention relates to a handheld eye wash apparatus used for first aid where there is a need for immediate washing of the eye, caused by foreign objects or chemicals in and/or around the eye(s).

In a first aspect the invention relates to an apparatus (**1**) for eye washing, where the apparatus comprises two rocker arms (**2**), which are hinged at a hinge (**3**), where each of the rocker arms on one side of the hinge has a jaw (**4**) for placing on the eyelids, and a wing (**5**) on the opposite side of the hinge, thereby by manual compression of the wings, and the thereby caused rotation of the rocker arms around the hinge, to force the jaws and thus the eyelids apart, where the apparatus comprises a connecting part (**6**) for connection to a liquid container (**13**). The rocker arms are connected to a pressure device (**9**) configured by compression of the wings to press a pressure valve (**14**) on the liquid container being a pressurized container of aerosol type (**13**) with a pressure valve (**14**), and thereby cause the release of liquid from the liquid container.

In a further aspect the pressure valve (**14**) comprises a springy mounted cylindrical nozzle tube (**15**) in extension of the container, thereby pressing the cylindrical nozzle tube into the container to release liquid from the container through the cylindrical nozzle tube. Thereby the springy mounted cylindrical nozzle contributes to the wings going back to an uncompressed condition after termination of the manual compression of the wings.

In a further aspect the rocker arms (**2**) are made in one piece connected (**23**) substantial at the level of the hinge (**3**) providing an elastic hinge effect between the rocker arms, thereby the elastic hinge effect contributes to the wings going back to an uncompressed condition after termination of the manual compression of the wings.

In a further aspect each jaw has a concave contact part (**10**) to the curved placing on the eyelid. The contact part (**10**) may be made of a resilient plastic material conforming to the shape of the eyelid contour.

In a further aspect each of the jaws besides a contact part has a connecting part (**11**) between the contact part (**10**) and the hinge (**3**), where the connecting part is more rigid than the contact part, thereby ensure sufficiently stiffness of the jaw to force and keep the eyelids apart, especially when the contact part is made of a resilient material.

In a further aspect the pressure device comprises a first body (**16**) supported by the nozzle tube (**15**), for pressing the nozzle tube in the direction of the container, and a second body constituting a shroud (**17**) around the nozzle tube (**15**) which shroud is provided with radially outward extending shoulders (**18**) for interaction with the wings (**5**), which shoulders during compression of the wings interacts with the first body (**16**) to press the first body and the nozzle tube towards the container.

In a further aspect each of the shoulders (**18**) has an outwardly extending, inclining surface (**19**).

In a further aspect the wings (**5**) comprises a pair, in relation to the nozzle tube (**15**), inclined contact faces (**20**), which by compression of the wings are in sliding interaction with the inclining surfaces (**19**) of the shoulders (**18**) when the compression of the wings to press the shoulders and thereby pressing the nozzle tube in direction of the container. The inclining contact faces may be convex curved.

In a further aspect each of the shoulders (**18**) of the shroud are hinged to the shroud at a flexible hinge (**21**).

In a further aspect the second body (**17**) is provided with stiffening parts (**22**) supporting the hinged one piece jaws to the hinge (**3**).

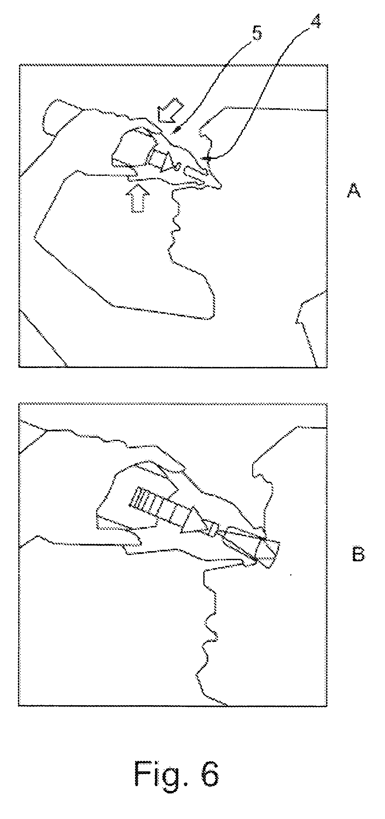

In a further aspect the apparatus is suitable for operation with one hand (**fig. 6** **A and B**) by the manual compression of the wings (**5**) when placing the jaws (**4**) of the apparatus on the eyelids. Thus the apparatus is placed with the jaws on the eyelids; and the wings are compressed at each opposite side of the hinge (**3**), thereby causing the rotation of the rocker arms around the hinge to force the jaws and thus the eyelids apart, activating the valve (**14**) to release the liquid from the container (**13**), and washing the eye. This operation may be performed with one hand throughout the washing procedure.

The time duration from onset of washing the eye(s) to the wash is sufficient for the specific purpose varies. However typical times are in the interval 10-20 minutes without changing the container. Sufficient wash is obtained by spraying 10-20 ml liquid per minute, thus the liquid capacity of the container is preferable 100-500 ml, more preferable 200-300 ml. and most preferable about 250 ml.

The liquid in the container can be any liquid suitable for eye wash purpose. One example is an isotone (0.9%) sodium chloride solution.

The apparatus may be made of polypropylene.

Thus in a very preferred embodiment and best mode to carry out the invention, the apparatus (**1**) for eye washing comprises two rocker arms (**2**), which are hinged at a hinge (**3**), where each of the rocker arms on one side of the hinge has a jaw (**4**) for placing on the eyelids, and a wing (**5**) on the opposite side of the hinge, thereby by manual compression of the wings, and the thereby caused rotation of the rocker arms around the hinge, to force the jaws and thus the eyelids apart, where the apparatus comprises a connecting part (**6**) for connection to a liquid container (**13**). The rocker arms are connected to a pressure device (**9**) configured by compression of the wings to press a pressure valve (**14**) on the liquid container being a pressurized container of aerosol type (**13**) with a pressure valve (**14**), and thereby cause the release of liquid from the liquid container. The pressure valve (**14**) comprises a springy mounted cylindrical nozzle tube (**15**) in extension of the container, thereby pressing the cylindrical nozzle tube into the container to release liquid from the container through the cylindrical nozzle tube. Thereby the springy mounted cylindrical nozzle contributes to the wings going back to an uncompressed condition after termination of the manual compression of the wings. The rocker arms (**2**) are made in one piece connected (**23**) substantial at the level of the hinge (**3**) providing an elastic hinge effect between the rocker arms, thereby the elastic hinge effect contributes to the wings going back to an uncompressed condition after termination of the manual compression of the wings. Each jaw has a concave contact part (**10**) to the curved placing on the eyelid. The contact part (**10**) may be made of a resilient plastic material conforming to the shape of the eyelid contour. Each of the jaws has besides a contact part a connecting part (**11**) between the contact part (**10**) and the hinge (**3**), where the connecting part is more rigid than the contact part, thereby ensure sufficiently stiffness of the jaw to force and keep the eyelids apart, especially when the contact part is made of a resilient material. The pressure device comprises a first body (**16**) supported by the nozzle tube (**15**), for pressing the nozzle tube in the direction of the container, and a second body constituting a shroud (**17**) around the nozzle tube (**15**) which shroud is provided with radially outward extending shoulders (**18)** for interaction with the wings (**5**), which shoulders during compression of the wings interacts with the first body (**16**) to press the first body and the nozzle tube towards the container. Each of the shoulders (**18**) has an outwardly extending, inclining surface (**19**). The wings (**5**) comprises a pair, in relation to the nozzle tube (**15**), inclined contact faces (**20**), which by compression of the wings are in sliding interaction with the inclining surfaces (**19**) of the shoulders (**18)** when the compression of the wings to press the shoulders and thereby pressing the nozzle tube in direction of the container. The inclining contact faces may be convex curved. Each of the shoulders (**18**) of the shroud are hinged to the shroud at a flexible hinge (**21**). The second body (**17**) is provided with stiffening parts (**22**) supporting the hinged one piece jaws to the hinge (**3**).

The apparatus is suitable for operation with one hand **(****fig. 6** **A and B**) by the manual compression of the wings (**5**) when placing the jaws (**4**) of the apparatus on the eyelids. Thus the apparatus is placed with the jaws on the eyelids; and the wings are compressed at each opposite side of the hinge (**3**), thereby causing the rotation of the rocker arms around the hinge to force the jaws and thus the eyelids apart, activating the valve (**14**) to release the liquid from the container (**13**), and washing the eye. This operation is performed with one hand throughout the washing procedure.

## Claims

1. Apparatus (**1**) for eye washing, where the apparatus comprises two rocker arms (**2**), which are hinged at a hinge (**3**), where each of the rocker arms on one side of the hinge has a jaw (**4**) for placing on the eyelids, and a wing (**5**) on the opposite side of the hinge, thereby by manual compression of the wings, and the thereby caused rotation of the rocker arms around the hinge, to force the jaws and thus the eyelids apart, where the apparatus comprises a connecting part (**6**) for connection to a liquid container (**13**), **characterised in** the liquid container being a pressurized container of aerosol type (**13**) with a pressure valve (**14**), and **in that** the rocker arms are connected to a pressure device (**9**) configured by compression of the wings to press the pressure valve (**14**) and thereby cause the release of liquid from the liquid container.

2. An apparatus according to claim 1 with a liquid container of aerosol type (**13**), where the liquid container is a container with pressurized liquid and a pressure valve **(14**) with a springy mounted cylindrical nozzle tube (**15**) in extension of the container, thereby pressing the cylindrical nozzle tube into the container to release liquid from the container through the cylindrical nozzle tube.

3. An apparatus according to the claims 1 or 2, where the rocker arms (**2**) are made in one piece connected (**23**) substantial at the level of the hinge (**3**) providing an elastic hinge effect between the rocker arms, thereby the elastic hinge effect contributes to the wings going back to an uncompressed condition after termination of the manual compression of the wings.

4. An apparatus according to any of the preceding claims, where each jaw has a concave contact part (**10**) to the curved placing on the eyelid.

5. An apparatus according to claim 4, where the contact part (**10**) is made of a resilient plastic material conforming to the shape of the eyelid contour.

6. An apparatus according to the claims 4 or 5, where each of the jaws besides a contact part has a connecting part (**11**) between the contact part (**10**) and the hinge (**3**), where the connecting part is more rigid than the contact part.

7. An apparatus according to claim 6, where the pressure device comprises a first body (**16**) supported by the nozzle tube (**15**), for pressing the nozzle tube in the direction of the container, and a second body constituting a shroud (**17**) around the nozzle tube (**15**) which shroud is provided with radially outward extending shoulders (**18**) for interaction with the wings (**5**), which shoulders during compression of the wings interacts with the first body (**16**) to press the first body and the nozzle tube towards the container.

8. An apparatus according to claim 7, where each of the shoulders (**18**) has an outwardly extending, inclining surface (**19**).

9. An apparatus according to claim 7 or 8, where the wings (**5**) comprises a pair, in relation to the nozzle tube (**15**), inclined contact faces (**20**), which by compression of the wings are in sliding interaction with the inclining surfaces (**19**) of the shoulders (**18**) when the compression of the wings to press the shoulders and thereby pressing the nozzle tube in direction of the container.

10. An apparatus according to claim 9, where the inclining contact faces (**20**) are convex curved.

11. An apparatus according to any of the claims 7-10, where each of the shoulders (**18**) of the shroud are hinged to the shroud at a flexible hinge (**21**).

12. An apparatus according to any of the claims 7-11, where the second body (**17**) is provided with stiffening parts (**22**) supporting the hinged one piece jaws to the hinge (**3**).

13. An apparatus according to any of the preceding claims where the apparatus is configured for operation with one hand by the manual compression of the wings when placing the jaws of the apparatus on the eyelids.

## Patentansprüche

1. Gerät (1) zum Augenspülen, wobei das Gerät zwei um ein Scharnier (3) drehbare Schwenkarme (2) aufweist, wobei jeder Schwenkarm an einer Scharnierseite eine Backe (4) zum Anbringen auf die Augenlider sowie einen Flügel (5) an der gegenüberliegenden Seite des Scharniers aufweist, so dass durch manuelles Zusammendrücken der Flügel und das daraus folgende Schwenken der Schwenkarme um das Scharnier ein Auseinanderzwingen der Backen und somit der Augenlider hervorgerufen wird, wobei das Gerät einen Verbindungsteil (6) zur Verbindung mit einem Flüssigkeitsbehälter (13) umfasst, **dadurch gekennzeichnet, dass** der Flüssigkeitsbehälter ein mit einem Druckventil (14) ausgestatteter, unter Druck stehender Behälter des Aerosoltyps (13) ist, und dass die Schwenkarme mit einer durch Zusammendrücken der Flügel zum Drücken des Druckventils (14) gestalteten Druckeinrichtung (9) verbunden sind und dabei die Freigabe der Flüssigkeit vom Flüssigkeitsbehälter veranlassen.

2. Gerät nach Anspruch 1 mit einem Flüssigkeitsbehälter des Aerosoltyps (13), wobei der Flüssigkeitsbehälter ein Behälter ist mit unter Druck stehender Flüssigkeit und einem Druckventil (14) mit einem federartig angeordneten, sich in Verlängerung des Behälters erstreckenden, zylinderförmigen Düsenrohr (15), wobei das zylinderförmige Düsenrohr zur Freigabe der Flüssigkeit vom Flüssigkeitsbehälter durch das zylinderförmige Düsenrohr in den Behälter gedrückt wird.

3. Gerät nach Anspruch 1 oder 2, wobei die Schwenkarme (2) einstückig und im wesentlichen an der Ebene des Scharniers (3) verbunden (23) sind und eine elastische Scharnierwirkung zwischen den Schwenkarmen erzeugen, wobei die elastische Scharnierwirkung zur Zurückführung der Flügel in einen nicht zusammengedrücken Zustand nach dem Aufhören des manuellen Zusammendrückens der Flügel beiträgt.

4. Gerät nach irgendeinem der vorhergehenden Ansprüche, wobei jede Backe einen konkaven Berührungsteil (10) zum gekrümmten Anbringen gegen das Augenlid aufweist.

5. Gerät nach Anspruch 4, wobei der Berührungsteil (10) aus einem federnden, sich der Form der Augenlidkontur anpassenden Kunststoffmaterial hergestellt ist.

6. Gerät nach Anspruch 4 oder 5, wobei jede Backe ausser einem Berührungsteil auch einen Verbindungsteil (11) zwischen dem Berührungsteil (10) und dem Scharnier (3) aufweist, wobei der Verbindungsteil steifer ist als der Berührungsteil.

7. Gerät nach Anspruch 6, wobei die Druckeinrichtung zum Drücken des Düsenrohrs in Richtung des Behälters einen ersten, durch das Düsenrohr (15) unterstützten Körper (16) aufweist, sowie einen zweiten Körper, der eine Hülle (17) um das Düsenrohr (15) bildet, wobei die Hülle zum Interagieren mit den Flügeln (5) radial nach außen verlaufende Schultern (18) aufweist, wobei die Schultern während des Zusammendrückens der Flügel zum Drücken vom ersten Körper und dem Düsenrohr gegen den Behälter mit dem ersten Körper (16) interagieren.

8. Gerät nach Anspruch 7, wobei die Schultern (18) jeweils eine nach außen verlaufende, sich neigende Fläche (19) aufweisen.

9. Gerät nach Anspruch 7 oder 8, wobei die Flügel (5) ein Paar relativ zum Düsenrohr (15) sich neigende Berührungsflächen (20) aufweisen, die durch Zusammendrücken der Flügel in gleitender Interaktion mit den sich neigenden Flächen (19) der Schultern (18) sind, wenn das Zusammendrücken der Flügel zum Drücken der Schultern und somit dem Drücken des Düsenrohrs in Richtung des Behälters.

10. Gerät nach Anspruch 9, wobei die sich neigenden Berührungsflächen (20) konvex gekrümmt sind.

11. Gerät nach irgendeinem der Ansprüche 7-10, wobei die Schultern (18) der Hülle jeweils an einem elastischen Scharnier (21) mit der Hülle schwenkbar angeordnet sind.

12. Gerät nach irgendeinem der Ansprüche 7-11, wobei der zweite Körper (17) mit Versteifungsteilen (22) versehen ist, die die schwenkbar angeordneten, einstückigen Backen zum Scharnier (3) unterstützen.

13. Gerät nach irgendeinem der vorhergehenden Ansprüche, wobei das Gerät zur Einhandbedienung durch manuelles Zusammendrücken der Flügel beim Anbringen der Gerätebacken gegen die Augenlider gestaltet ist.

## Revendications

1. Appareil (1) pour le lavage des yeux, l'appareil comprenant deux bras culbuteurs (2) articulés par une charnière (3), chacun des bras culbuteurs d'un côté de la charnière comprend une mâchoire (4) destinée à être positionnée sur les paupières, et une aile (5) sur le côté opposé de la charnière, par compression manuelle des ailes, et la rotation engendrée ainsi des bras culbuteurs autour de la charnière, pour forcer les mâchoires et les paupières à s'écarter les unes des autres, l'appareil comprenant une pièce de raccordement (6) pour raccordement à un conteneur de liquide (13), **caractérisé en ce que** le conteneur de liquide constitue un conteneur sous pression du type aérosol (13) pourvu d'une soupape de pression (14), et que les bras culbuteurs sont raccordés à un dispositif de pression (9) configuré par la compression des ailes pour pousser la soupape de pression (14), et ainsi entraîner libération du liquide depuis le conteneur de liquide.

2. Appareil selon la revendication 1 comprenant un conteneur de liquide du type aérosol (13), le conteneur de liquide constitue un conteneur contenant du liquide sous pression et une soupape de pression (14) comprenant un tube de buse cylindrique monté de façon flexible (15) qui s'étende dans la longueur du conteneur, le tube de buse cylindrique est poussé dans le conteneur pour libérer du liquide du conteneur à travers le tube de buse cylindrique.

3. Appareil selon la revendication 1 ou 2, les bras culbuteurs (2) sont fabriqués en une seule pièce raccordée (23) principalement au niveau de la charnière (3) produisant un effet charnière et élastique entre les bras culbuteurs, ainsi l'effet charnière et élastique contribue à ce que les ailes reviennent à une condition non comprimée après la fin de la compression des ailes.

4. Appareil selon l'une quelconque des revendications précédentes, chacune des mâchoires comprend une partie de contact de forme concave (10) pour l'emplacement recourbé sur la paupière.

5. Appareil selon la revendication 4, la partie de contact (10) est composé d'un matériau plastique élastique adapté à la forme du contour de la paupière.

6. Appareil selon la revendication 4 ou 5, en plus d'une partie de contact, chacune des mâchoires comprend une pièce de raccordement (11) entre la partie de contact (10) et la charnière (3), où la pièce de raccordement est plus rigide que la partie de contact.

7. Appareil selon la revendication 6, le dispositif de pression comprend un premier corps (16) supporté par un tube de buse (15) pour pousser le tube de buse dans la direction du conteneur, et un deuxième corps comprenant un revêtement (17) autour du tube de buse (15), lequel revêtement est réalisé avec des épaulements (18) s'étendant radialement vers l'extérieur pour une interaction avec les ailes (5), lesquels épaulements interagissent avec le premier corps (16) pendant la compression des ailes afin de pousser le premier corps et le tube de buse vers le conteneur.

8. Appareil selon la revendication 7, chacun des épaulements (18) comprend une surface (19) inclinée s'étendant vers l'extérieur.

9. Appareil selon la revendication 7 ou 8, les ailes (5) comprennent une paire de surfaces de contact inclinées (20) vis-à-vis du tube de buse (15) qui, par compression des ailes, sont en interaction de manière glissante avec les surfaces inclinées (19) des épaulements (18) au moment de la compression des ailes pour presser les épaulements et ainsi le tube de buse dans la direction du conteneur.

10. Appareil selon la revendication 9, les surfaces de contact inclinées (20) présentent une courbure convexe.

11. Appareil selon l'une quelconque des revendications 7 à 10, chacun des épaulements (18) du revêtement est articulé au revêtement avec une charnière souple (21).

12. Appareil selon l'une quelconque des revendications 7 à 11, le deuxième corps (17) comporte des éléments raidisseurs (22) servant de support pour les mâchoires en une pièce et articulées à la charnière (3).

13. Appareil selon l'une quelconque des revendications précédentes, où l'appareil est configuré pour une utilisation avec une seule main en compressant les ailes de façon manuelle au moment où les mâchoires de l'appareil sont positionnées sur les paupières.
